(19) Europäisches Patentamt
European Patent Office
Office européen des brevets

(11) Veröffentlichungsnummer: **0 032 661**
**A1**

(12) **EUROPÄISCHE PATENTANMELDUNG**

(21) Anmeldenummer: **81100031.4**

(22) Anmeldetag: **07.01.81**

(51) Int. Cl.³: **C 07 C 143/70**, C 07 C 143/29 // C07F9/40

(30) Priorität: **19.01.80 DE 3001876**

(43) Veröffentlichungstag der Anmeldung: **29.07.81 Patentblatt 81/30**

(84) Benannte Vertragsstaaten: **CH DE FR GB IT LI**

(71) Anmelder: **BAYER AG, Zentralbereich Patente, Marken und Lizenzen, D-5090 Leverkusen 1, Bayerwerk (DE)**

(72) Erfinder: **Bremen, Josef, Dr., Am Kettnersbusch 13, D-5090 Leverkusen 3 (DE)**
Erfinder: **Dorlars, Alfons, Dr., Mozartstrasse 32, D-5090 Leverkusen 1 (DE)**
Erfinder: **Schellhammer, Carl-Wolfgang, Dr., Katharinenthal 8, D-5060 Bergisch-Gladbach 2 (DE)**
Erfinder: **Wehling, Bernhard, Dr., Andreas-Gryphius-Strasse 26, D-5000 Koeln 80 (DE)**

(54) **Verfahren zur Herstellung von Styrylsulfonsäurederivaten.**

(57) Gegebenenfalls weiter-substituierte Styrylverbindungen der Formel

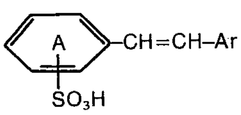

(Ar = Aryl)

werden in einfacher Weise dadurch erhalten, daß man Verbindungen der Formel

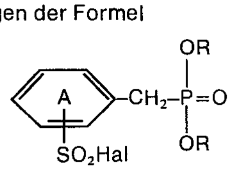

(R = KW-Rest)

mit Aldehyden der Formel

Ar–CHO

in Gegenwart starker Basen kondensiert. Die neuen Verbindungen sind Ausgangsstoffe zur Herstellung wertvoller optischer Aufheller vom Stilben-Typ.

EP 0 032 661 A1

BAYER AKTIENGESELLSCHAFT 5090 Leverkusen, Bayerwerk
Zentralbereich
Patente, Marken und Lizenzen K/kl-c

Verfahren zur Herstellung von Styrylsulfonsäurederivaten

Gegenstand der Erfindung ist ein verbessertes Verfahren zur Herstellung teilweise bekannter Styrylverbindungen, die in Form der freien Säure der Formel

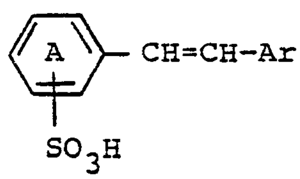

(I)

entsprechen, worin der Ring A und der Arylrest Ar durch in der Chemie der optischen Aufheller übliche Substituenten substituiert sein können.

Das neue Verfahren ist dadurch gekennzeichnet, daß man Verbindungen der Formel

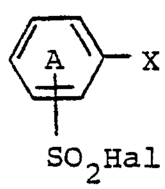

(II)

worin

A die obengenannte Bedeutung hat,
Hal für Halogen und
X für eine phosphoraktivierte Methylengruppe stehen,

mit Aldehyden der Formel

Ar-CHO (III)

worin Ar die obengenannte Bedeutung hat,

in Gegenwart starker Basen in an sich bekannter Weise kondensiert.

Diese Kondensation basiert auf dem Prinzip der Wittig-Horner-Synthese und wird im wesentlichen unter den für diese Synthese üblichen Bedingungen durchgeführt, d.h. in praktisch wasserfreien polaren organischen Lösungsmitteln bei Temperaturen von 20 - 80°C in Gegenwart basischer Kondensationsmittel, wie Alkalialkoholate und -hydroxide sowie - in besonderen Fällen - Lithiumphenyl oder Trialkylammoniumhydroxide. Bevorzugt sind Alkalialkoholate, wie Natriummethylat.

Dennoch ist der Reaktionsablauf als überraschend zu bezeichnen, da erwartet werden konnte, daß unter den vorstehend beschriebenen Bedingungen die Sulfonsäureester oder -amide entstehen und nicht die Sulfonsäuren bzw. deren Salze.

Von den basischen Kondensationsmitteln werden mindestens 4 Mol pro Mol Verbindung II eingesetzt.

Die Reaktionszeiten liegen zwischen 30 Minuten und mehreren Stunden.

Weitere präparative Einzelheiten zur Durchführung dieser Reaktion findet man in DE-OS 2 525 681, 2 525 684 und 1 923 267.

Geeignete "phosphoraktivierte" Methylengruppen X sind solche der Formeln

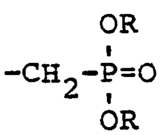 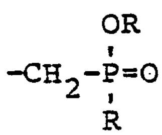 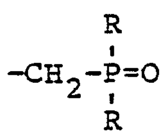

- im weitesten Sinne auch - 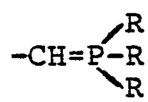 .

Technisch interessant ist jedoch allein die erstgenannte Gruppierung.

Als Reste R kommen Alkyl, Cycloalkyl, Aralkyl oder Aryl in Betracht. Geeignete Alkylreste R sind $C_1$-$C_4$-Alkyl; geeignete Cycloalkylreste R sind Cyclohexylreste; geeignete Aralkylreste R sind Benzylreste und geeignete Arylreste sind Phenylreste. Diese Reste können prinzipiell weitere Substituenten tragen, was technisch jedoch wenig sinnvoll ist, da sie bei der Reaktion abgespalten werden und nicht ins Endprodukt eingehen.

Bevorzugte Verfahrensprodukte sind solche der Formel

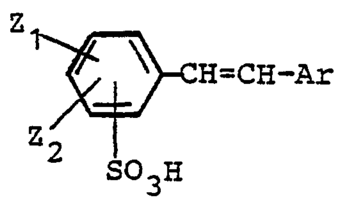

(IV)

worin

Ar für Phenyl, Naphthyl oder Biphenyl,

$Z_1$ für Wasserstoff, Alkyl, Benzyl, Phenyl, Styryl, $CF_3$, Halogen, Alkoxy, Alkoxycarbonyl, Alkylcarbonylamino, Phenylcarbonylamino, Alkylsulfonyl, Phenylsulfonyl oder X,

$Z_2$ für Wasserstoff, Alkyl oder Alkoxy und

$Z_1$ mit $Z_2$ gemeinsam für den Rest $-(CH_2)_3-$, $-(CH_2)_4-$ oder -CH=CH-CH=CH- stehen,

wobei die genannten Alkyl- und Alkoxyreste 1 - 4 C-Atome aufweisen und die genannten aromatischen Reste z.B. durch Alkyl, $SO_3H$, $NO_2$, CHO, COOH, Alkylcarbonylamino, Alkoxy oder Halogen substituiert sein können.

Geeignete Halogensubstituenten sind F, Br und vor allem Cl.

Besonders bevorzugte Verfahrensprodukte sind solche der Formel

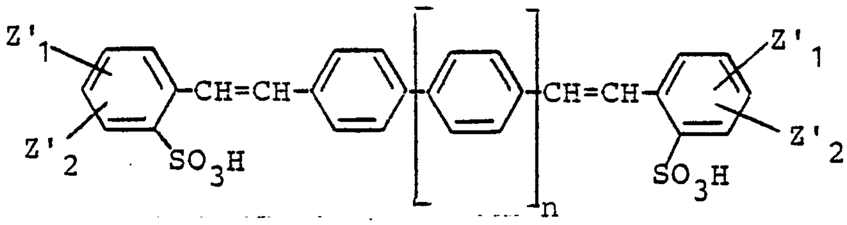

worin $Z'_1/Z'_2$ Wasserstoff, Chlor, Methyl oder $(CH_2)_4$ und

n O oder 1

bedeuten.

Die in dem beanspruchten Verfahren eingesetzten Ausgangsmaterialien der Formel (II) sind bislang nicht in der Literatur beschrieben worden. Man erhält diese jedoch relativ leicht, indem man Verbindungen der Formel

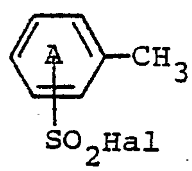

(V)

in an sich bekannter Weise halogeniert und die Reaktionsprodukte der Formel

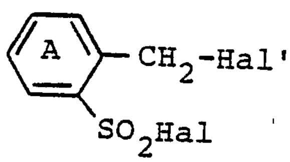

(VI)

wobei Hal für F steht, und Hal' für Halogen, vorzugsweise Cl oder Br steht, beispielsweise mit Phosphiten der Formel

$$P(OR)_3 \qquad (VII)$$

bei 110 - 180°C nach Art einer Michaelis-Arbusov Reaktion zu den Verbindungen II umsetzt (vgl. Houben-Weyl, 4. Aufl., Bd. XII/1, S. 433 ff).

Die Verfahrensprodukte der Formel (I) sind z. T. bekannte wertvolle optische Aufheller, insbesondere für Cellulosefasermaterialien (vgl. die oben erwähnte Patentliteratur sowie DE-PS 1 794 386 und 1 793 482 und DE-OS 19 23 267).

Diese Verbindungen wurden bislang in der Weise hergestellt, daß man Verbindungen der Formel

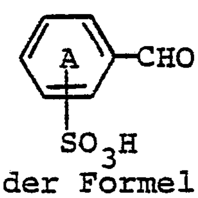

mit Verbindungen der Formel

Ar-X

kondensierte.

Nachteilig an dieser Methode ist indessen die Tatsache, daß die zur Herstellung der Verbindungen Ar-X benötigten Verbindungen $Ar-CH_2-Hal$ (Hal vorzugsweise = Cl) im technischen Maßstabe nur durch Chlormethylierung der Grundkohlenwasserstoffe Ar-H zugänglich sind, wobei hochtoxische Nebenprodukte entstehen, deren Handhabung bzw. Beseitigung einen erheblichen apparativen Aufwand erfordert.

Diese Nachteile weist das erfindungsgemäße Verfahren nicht auf, so daß es eine wesentliche Bereicherung der Technik darstellt.

Das neue Verfahren sei an Hand der folgenden Beispiele näher erläutert.

Beispiel 1

37,2 g o-Fluorsulfonylbenzylphosphonsäurediethylester (92,7 %ig) und 6,7 g Terephthalaldehyd werden in 75 ml trockenem N,N-Dimethylformamid unter Stickstoff bei 30 - 45°C mit 90 g 30 %iger Natriummethylat-Lösung versetzt. Man rührt anschließend 5 Stunden bei 65°C und trägt das Reaktionsgemisch auf 350 ml heißes Wasser aus. Die Lösung wird neutralisiert und mit 350 ml gesättigter Kochsalzlösung sowie 100 g Kochsalz versetzt. Das abgeschiedene gelbe Material wird nach 2 Stunden abgesaugt, an der Luft getrocknet und unter Zusatz von A-Kohle umgelöst. Man erhält 2',2"-Disulfodistyrylbenzol als gelbe Kristalle. Das Absorptionsmaximum einer wäßrigen Lösung der Substanz liegt bei 350,3 m, die Extinktion beträgt 51 800.

Wenn man anstelle von Terephthalaldehyd den Biphenyl-4,4'-Dicarboxaldehyd verwendet, erhält man 4,4'-Bis-(2"-sulfostyryl)-biphenyl, $\lambda_{max}$= 348,2 µ, $\varepsilon_{max}$ = 64 200.

Beispiel 2

6,7 g Terephthalaldehyd und 32,7 g p-Chlorsulfonylphenylphosphonsäurediethylester in 100 ml trockenem N,N-Dimethylformamid werden unter Stickstoff bei 30 - 40°C in 1/2 Stunde mit 144 g 30 %iger Natriummethylatlösung versetzt. Man rührt die Mischung 5 Stunden bei 65°C, trägt sie auf 350 ml heißes Wasser aus, stellt mit konzentrierter Salzsäure neutral und versetzt mit 350 ml gesättigter Kochsalzlösung. Am nächsten Tage wird das Produkt abgesaugt und mit einer Mischung aus 250 ml konzentrierter Salzsäure und 250 ml Wasser 1 Stunde rückfließend gekocht. Das Produkt wird abgesaugt, in 2 Liter Wasser aufgenommen, mit Kaliumcarbonat schwach alkalisch gestellt, aufgekocht und filtriert. Aus dem Filtrat scheidet sich das Kaliumsalz des 1,4-Bis-(p-sulfostyryl)-benzols in Gestalt von hellgelben Kriställchen ab. Das Absorptionsmaximum der Verbindung liegt bei 343,6 mμ. ($\varepsilon_{max}$ = 38 900).

Beispiel 3

4,6 g Pyren-3-aldehyd und 7,5 g o-Fluorsulfonylbenzylphosphonsäurediethylester werden in 30 ml trockenem N,N-Dimethylformamid unter Stickstoff bei 30 - 45°C in 1/4 Stunde mit 18 g 30 %iger Natriummethylatlösung versetzt. Man rührt die Mischung 5 Stunden bei 65°C, trägt auf 350 ml heißes Wasser aus, neutralisiert mit Salzsäure und salzt das Produkt mit 350 ml gesättigter Kochsalzlösung aus. Die eigelben Kristalle werden isoliert und aus Wasser umgelöst. Das Natriumsalz des 3-o-Sulfostyrylpyrens kristallisiert mit einem Kristallwasser. $\lambda_{max}$ = 373,1 mμ, $\varepsilon_{max}$ = 37 100.

Die Verbindung hellt nach wie üblich durchgeführter Applikation Polyamid auf.

Beispiel 4

5,5 g 4-⟨Naphtho[1,2:d]triazolyl-(2)⟩-benzaldehyd und 6,6 g 4-Fluorsulfonylbenzylphosphonsäurediethylester werden in 50 ml trockenem N,N-Dimethylformamid bei Raumtemperatur mit 18 g 30 %iger Natriummethylatlösung versetzt. Man rührt die Mischung 2 Stunden bei 20 - 30°C und 2 Stunden bei 65°C, dann neutralisiert man mit 6 ml Eisessig und dampft zur Trockene ein. Den Rückstand kocht man mit 100 ml Wasser auf, kühlt ab, saugt das abgeschiedene Produkt ab und löst es aus N,N-Dimethylformamid/Wasser um. Man erhält das Natriumsalz der 4-⟨Naphtho[1,2:d]-triazol-(2)⟩-stilben-4'-sulfonsäure als hellgelbe Kriställchen. ($\lambda_{max}$ = 361,3 mμ. $\varepsilon_{max}$ = 33 500).

Beispiel 5

6,4 g Benzaldehyd, 17,3 g Fluorsulfonylbenzol-2,5-bismethanphosphonsäurediethylester und 50 ml N,N-Dimethylformamid werden unter Stickstoff mit 27 g 30 %iger Natriummethylatlösung versetzt. Die Mischung wird 2 Stunden bei Raumtemperatur und 2 Stunden bei 65°C gerührt und dann analog wie in Beispiel 4 angegeben aufgearbeitet. Nach dem Umlösen aus Wasser erhält man 2,5-Distyrylbenzolsulfonsäure als Natriumsalz in Gestalt von farblosen Kriställchen ($\lambda_{max}$ = 350,9 mμ, $\varepsilon_{max}$ = 48 600).

Beispiel 6

4,1 g 4-Diethoxyphosphonomethyl-biphenyl-4'-sulfofluorid (94 %ig) werden mit 3,3 g 4-Formyl-biphenyl-4'-sulfonsaurem Natrium (94 %ig) bei Raumtemperatur in 28 ml N,N-Dimethylformamid gut verrührt. Man trägt darauf unter weiterem Rühren allmählich 6 g Kalium-tert.-butanolat (ca. 95 %ig) ein. Durch Kühlung sorgt man dafür, daß die Temperatur nicht über 40°C steigt, verdünnt dann mit 150 ml Wasser, stellt mit Salzsäure neutral und saugt das helle Stilbenderivat ab, das man durch Umlösen aus Methylglykol/Wasser reinigt. Ausbeute: 5,2 g. Helles, in Wasser ziemlich schwer lösliches Kristallpulver, das in N,N-Dimethylformamid im UV-Licht stark rotstichig-blau fluoresziert.

Beispiel 7

2,7 g (0,02 Mol) Terephthalaldehyd, 14,6 g (0,04 Mol) 4-Chlor-2-fluorsulfonylbenzylphosphonsäurediäthylester und 50 ml trockenes N,N-Dimethylformamid werden bei Raum=temperatur unter Stickstoff in 1/4 Stde. mit 36 g (0,2 Mol) 30%iger Natriummethylatlösung versetzt. Man rührt die Mi=schung noch 2 Stdn. bei Raumtemperatur und dann 2 Stdn. bei 65°. Nach dem Neutralisieren mit Eisessig engt man zur Trockene ein und löst den verbliebenen Rückstand aus Wasser um. Man erhält das Natriumsalz des 1,4-Bis-(4-chlor-2-sulfo=styryl)-benzols als gelbes Kristallisat( $_{max}$=28 150 $cm^{-1}$, $_{max}$=355,2 nm, $_{max}$=55 500).

Patentansprüche

1. Verfahren zur Herstellung von Styrylverbindungen, die in Form der freien Säure der Formel

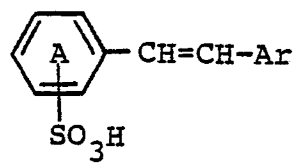

entsprechen, worin der Ring A und der Arylrest Ar durch in der Chemie der optischen Aufheller übliche Substituenten substituiert sein können, dadurch gekennzeichnet, daß man Verbindungen der Formel

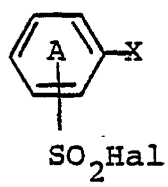

worin

A die obengenannte Bedeutung hat,
Hal für Halogen und
X für eine phosphoraktivierte Methylengruppe stehen,

mit Aldehyden der Formel

Ar-CHO

worin Ar die obengenannte Bedeutung hat,

in Gegenwart starker Basen in an sich bekannter Weise kondensiert.

2. Verfahren gemäß Anspruch 1, dadurch gekennzeichnet, daß man solche phosphorhaltigen Verbindungen der angegebenen Formel einsetzt, worin

X für $-CH_2-\overset{OR}{\underset{OR}{P}}=O$ steht,

wobei R die genannte Bedeutung hat.

Europäisches Patentamt

# EUROPÄISCHER RECHERCHENBERICHT

0032661

Nummer der Anmeldung
EP 81 10 0031

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CH - A - 505 036 (CIBA-GEIGY)<br>* Spalte 3, Zeilen 14-17 *<br>--- | 1,2 |
| P | EP - A - 0 009 679 (BAYER)<br>* Insgesamt *<br>-------- | 1,2 |

KLASSIFIKATION DER ANMELDUNG (Int. Cl.³)

C 07 C 143/70
143/29//
C 07 F 9/40

RECHERCHIERTE SACHGEBIETE (Int. Cl.³)

C 07 F 9/40
C 07 C 143/70
143/29

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 06-04-1981 | GRAMAGLIA |

EPA form 1503.1 06.78